(19) **Europäisches Patentamt / European Patent Office / Office européen des brevets**

(11) **EP 4 317 173 A1**

(12) **EUROPEAN PATENT APPLICATION**
published in accordance with Art. 153(4) EPC

(43) Date of publication:
**07.02.2024 Bulletin 2024/06**

(21) Application number: **22775766.3**

(22) Date of filing: **24.03.2022**

(51) International Patent Classification (IPC):
$C07K\ 7/06^{(2006.01)}$    $A23L\ 33/18^{(2016.01)}$
$A61K\ 38/08^{(2019.01)}$    $A61P\ 25/20^{(2006.01)}$
$A61P\ 25/22^{(2006.01)}$    $A61P\ 25/24^{(2006.01)}$
$A61P\ 25/28^{(2006.01)}$    $C07K\ 1/12^{(2006.01)}$
$C12P\ 21/06^{(2006.01)}$

(52) Cooperative Patent Classification (CPC):
**A23L 33/18; A61K 38/08; A61P 25/20;
A61P 25/22; A61P 25/24; A61P 25/28; C07K 7/06;**
C07K 1/12; C12P 21/06

(86) International application number:
**PCT/JP2022/013912**

(87) International publication number:
**WO 2022/202985 (29.09.2022 Gazette 2022/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.03.2021   JP 2021050468**

(71) Applicants:
• **Morinaga Milk Industry Co., Ltd.
Minato-ku
Tokyo 108-8384 (JP)**
• **Kyoto University
Kyoto-shi, Kyoto 606-8501 (JP)**

(72) Inventors:
• **OHINATA Kousaku
Kyoto-shi, Kyoto 606-8501 (JP)**
• **FUJII Hikaru
Kyoto-shi, Kyoto 606-8501 (JP)**
• **NAKADA Hajime
Zama-shi, Kanagawa 252-8583 (JP)**
• **KURIMOTO Masaki
Zama-shi, Kanagawa 252-8583 (JP)**
• **OCHI Hiroshi
Zama-shi, Kanagawa 252-8583 (JP)**

(74) Representative: **Hoffmann Eitle
Patent- und Rechtsanwälte PartmbB
Arabellastraße 30
81925 München (DE)**

Remarks:
The complete document including Reference Table(s) and the Sequence Listing(s) can be downloaded from the EPO website

(54) **PEPTIDE AND COMPOSITION CONTAINING PEPTIDE AS ACTIVE INGREDIENT**

(57)    A peptide which can be easily taken on a daily basis and which has few side effects and has various actions and a composition containing the peptide as an active ingredient are provided. A peptide having the amino acid sequence of (a) or (b) below is provided. (a) The amino acid sequence of SEQ ID NO: 1 (Asn-Leu-Pro-Pro-Leu-Thr). (b) An amino acid sequence in which one or several amino acids are substituted, deleted or added in the amino acid sequence of SEQ ID NO: 1. Moreover, a composition containing the peptide as an active ingredient is also provided.

EP 4 317 173 A1

**Description**

Technical Field

The present invention relates to a peptide and a composition containing the peptide as an active ingredient.

Background Art

Recently, as a result of various types of stress due to diversification of living environments and working environments, complicated human relations and the like, many people living in modern society are exposed to many feelings of uneasiness or psychological stress. When exposed to such feelings of uneasiness or psychological stress for a long period of time, disorders of physical health, psychiatric disorders, sleep disorders, learning disorders, memory impairment or the like sometimes develop. When such a disorder develops, long-term treatment with a medication having side effects is sometimes required, and it is thus necessary to appropriately relieve the feelings of uneasiness or the psychological stress.

Use of chemically synthesized drugs such as tranquilizers, sleep-inducing drugs, sleep aids and antidepressants for controlling the stress on a daily basis, however, is not necessarily appropriate in view of the side effects and the like. Accordingly, a material which can be easily taken on a daily basis and which has few side effects is required.

PTL 1 discloses a peptide containing tyrosine, phenylalanine, tryptophan or histidine and a hydrophobic amino acid adjacent thereto as a peptide having an anxiolytic action. Moreover, PTL 2 discloses a peptide having any of (i) the amino acid sequence LSSTQAQQSY, (ii) the amino acid sequence LSSTQAQQSW and (iii) the amino acid sequence LSSTQAQQSF as a peptide which improves or treats diminished motivation, depression, depressive mood disorder or a symptom caused thereby.

However, because there are various requirements for a material having an anxiolytic action, a sleep-improving action, an antidepressive action or an action of improving learning disorder and/or memory impairment, there is still a demand for a novel material.

Citation List

Patent Literature

    PTL 1: WO2010/087480
    PTL 2: WO2016/140277

Summary of Invention

Technical Problem

Thus, a main aspect of the present technology is to provide a peptide which can be easily taken on a daily basis and which has few side effects and has various actions, and a composition containing the peptide as an active ingredient.

Solution to Problem

As a result of extensive investigation to solve the problem, the present inventors have found a peptide having the amino acid sequence Asn-Leu-Pro-Pro-Leu-Thr (SEQ ID NO: 1, also called "peptide NLPPLT" below), which has an anxiolytic action, a sleep-improving action, an antidepressive action and a learning disorder- and/or memory impairment-improving action and the inventors have completed the present technology as described herein.

That is, in the present technology, a peptide having the amino acid sequence of (a) or (b) below is first provided:

    (a) the amino acid sequence of SEQ ID NO: 1,
    (b) an amino acid sequence in which one or several amino acids are substituted, deleted or added in the amino acid sequence of SEQ ID NO: 1.

The peptide as described above may have an anxiolytic action, a sleep-improving action, an antidepressive action or a learning disorder- and/or memory impairment-improving action.

Moreover, in the present technology, a composition containing the peptide as described above as an active ingredient is also provided.

The composition as described above may be a food or a drink.

The composition as described above may be a pharmaceutical product. In this case, the composition as described herein may be used for treating, preventing and/or improving depression, bipolar disorder, schizophrenia, anxiety neurosis or sleep disorder.

Advantageous Effects of Invention

In the present technology, because the peptide NLPPLT has an excellent anxiolytic action, an excellent sleep-improving action, an excellent antidepressive action and an excellent action of improving learning disorder and/or memory impairment, can be easily taken on a daily basis and has few side effects and has various actions, a composition containing the peptide as an active ingredient can be provided.

In this regard, the effects described herein are not necessarily limited and may be any of the effects described in the present description.

Description of Embodiments

The embodiments explained below are examples of typical embodiments, and the scope should not be construed as being narrow by the embodiments.

1. Peptide

The peptide as described herein has the amino acid sequence represented by Asn-Leu-Pro-Pro-Leu-Thr (SEQ ID NO: 1). In the present technology, Asn (N) represents L-asparagine residue, and Leu (L) represents L-leucine residue. Pro (P) represents L-proline residue, and Thr (T) represents L-threonine residue.

Alternatively, the peptide as described herein has an amino acid sequence in which one or several amino acids are substituted, deleted or added in the amino acid sequence of SEQ ID NO: 1. In this case, the number of the substituted, deleted or added amino acids is preferably two or less.

Moreover, one or several amino acids may be substituted, deleted, or added in the peptide as described herein, in a range in which the peptide maintains any one or more actions selected from an anxiolytic action, a sleep-improving action, an antidepressive action and an action of improving learning disorder and/or memory impairment. Furthermore, the peptide as described herein may be a salt of the peptide NLPPLT. Examples of the salt include those of alkali metals such as potassium and sodium, and alkaline earth metals such as calcium and magnesium, and one kind or two or more kinds thereof can be appropriately used.

Regarding the method for producing the peptide NLPPLT, for example, the following method is exemplified, but the method is not particularly limited thereto. Specific examples thereof include a method of obtaining by hydrolyzing a protein or a peptide containing the amino acid sequence represented by Asn-Leu-Pro-Pro-Leu-Thr (SEQ ID NO: 1) by hydrolysis or the like and separating and/or purifying from the obtained hydrolyzed product, a method of obtaining by synthesizing the peptide NLPPLT by a chemical synthesis method of a peptide and then separating and/or purifying the peptide NLPPLT from the obtained composite, and a method of extracting from a plant, an animal or a microorganism which produces the peptide NLPPLT, a peptide containing the peptide NLPPLT or the like and separating and/or purifying from the obtained extract.

The peptide as described herein can be produced, for example, by hydrolyzing a protein such as whey protein appropriately with an acid, an alkali, an enzyme or the like. A method for obtaining the peptide NLPPLT by hydrolyzing a raw material protein with a protease is exemplified below.

First, before hydrolyzing a raw material protein with an enzyme, the protein is dissolved, dispersed or suspended in water.

The raw material protein is not particularly limited as long as the protein includes the peptide as described herein and can produce the peptide as described herein through appropriate hydrolysis with a protease. Examples of the protein include those derived from an animal, derived from a plant, and derived from a microorganism, and of these, whey protein, which can be obtained in a large amount, is preferable in the present technology.

The whey protein may be a commercial product, a whey protein concentrate obtained by separating and/or purifying from whey protein by a general method (for example, ultrafiltration, the ion exchange method or the like), isolated whey protein or a mixture of such whey protein, whey and the like (for example, whey powder, demineralized whey powder or the like) in which the purity of the whey protein is adjusted.

Here, the treatment method differs with the properties of the raw material protein. When the raw material protein is soluble, the raw material protein may be dispersed in water or warm water to dissolve the protein, and when the raw material protein is insoluble, the protein may be homogenized in hot water by mixing and stirring.

Then, an alkali agent or an acid agent may be added to the solution containing the protein to adjust the pH. The pH is preferably adjusted to the optimal pH of the protease used or around the optimal pH.

The alkali agent or the acid agent is not particularly limited, and those which are acceptable for a pharmaceutical product or a food or a drink may be used. Examples of the alkali agent include hydroxides such as sodium hydroxide and calcium hydroxide, and carbonates such as potassium carbonate, and these may be alkali metal salts or alkaline earth metal salts. Examples of the acid agent include inorganic acids such as hydrochloric acid and phosphoric acid, and organic acids such as citric acid, acetic acid and formic acid. In the present technology, one kind or two or more kinds thereof can be appropriately used.

Moreover, the solution containing the protein is preferably pasteurized at 70 to 90°C for around 15 seconds to 10 minutes to prevent deterioration caused by bacterial contamination.

Next, a certain amount of a protease is added to the solution containing the protein, and reaction is conducted at a temperature of around 10 to 85°C for 0.1 to 48 hours. Thus, a hydrolysate is obtained.

Here, after adding the protease, the solution is maintained at an appropriate temperature depending on the kind of the enzyme, for example at 30 to 60°C, preferably at 45 to 55°C, to start hydrolysis of the protein.

Regarding the hydrolysis reaction period, the reaction may be continued until a preferable hydrolyzed rate is achieved while monitoring the hydrolyzed rate of the enzymatic reaction.

The protease reaction is terminated, for example, by inactivating the enzyme in the hydrolysis solution and can be terminated by heat inactivation treatment by a general method. The heating temperature and the retention period of the heat inactivation treatment can be appropriately set to conditions for sufficient inactivation, considering the thermal stability of the enzyme used, but for example, the treatment can be conducted in a temperature range of 80 to 130°C for a retention time of 30 minutes to two seconds.

The protease is not particularly limited but is preferably an enzyme which can hydrolyze the raw material protein and produce the peptide as described herein. Moreover, one kind of protease alone or a combination of two or more kinds may be used. When two or more kinds of the enzyme are used, the enzymatic reactions may be conducted simultaneously or separately. As the enzyme which can produce, specifically, an endopeptidase, trypsin and papain are preferably used in combination, and a mixture of the three kinds of enzymes is more preferably used.

Examples of the endopeptidase include those derived from a microorganism and derived from an animal, and specific examples thereof include a protease derived from *Bacillus subtilus,* and a protease derived from an animal pancreas. As the protease, a commercial protease can be used. Examples of the commercial protease product include proteases derived from a bacterium of the genus *Bacillus* such as protease N Amano (manufactured by Amano Enzyme Inc.), Bioprase SP-20 (manufactured by Nagase & Co., Ltd.) and Neutrase (manufactured by Novozymes Japan Ltd.), and proteases derived from an animal pancreas such as PTN6.0S (manufactured by Novozymes Japan Ltd.).

Examples of the trypsin include PTN6.0S (manufactured by Novozymes Japan Ltd.), and examples of the papain include papain W-40 (manufactured by Amano Enzyme Inc.), and purified papain (manufactured by Mitsubishi Chemical Corporation).

In this regard, with respect to the method for calculating the degree of hydrolysis of the raw material protein, the total nitrogen amount of the sample is measured by the Kjeldahl method, and the formol nitrogen amount of the sample is measured by the formol titration method. The degree of hydrolysis is calculated from the measured values by the following equation (1).

[Math. 1]

$$\text{Degree of hydrolysis (\%) = (Formol Nitrogen Amount/Total Nitrogen Amount)} \times 100...(1)$$

In the present technology, the peptide as described herein is preferably isolated or purified from the liquid hydrolysate.

The peptide as described herein can be purified by an appropriate combination of methods similar to those which are generally used for purifying oligopeptides, for example, methods such as various types of chromatography including ion-exchange chromatography, adsorption chromatography, reversed-phase chromatography, partition chromatography, and gel-permeation chromatography, solvent precipitation, salt precipitation and partition between two types of liquid phase.

In isolating or purifying the peptide as described herein, a fraction containing the target substance can be determined using the actions described above as indicators. Moreover, the active ingredient of such a fraction can be identified by mass spectrometry.

The peptide as described herein can also be produced by chemical synthesis.

The peptide as described herein can be chemically synthesized by a liquid phase method or a solid phase method which is generally used for synthesizing oligopeptides. The peptide as described herein can be isolated by deprotecting the synthesized peptide according to the need and removing the unreacted reagent, the by-product or the like.

Such peptide synthesis can be conducted, for example, using a commercial peptide synthesizer or the like. To determine if the target peptide has been obtained can be examined using any one or more actions selected from an anxiolytic action, a sleep-improving action, an antidepressive action and an action of improving learning disorder and/or memory impairment as an indicator.

The peptide NLPPLT has an anxiolytic action as shown in Test Example 1 described below, and the action is achieved through activation of the serotonin 5-HT$_{1A}$ receptor as shown in Test Example 2 described below, and thus also has a sleep-improving action. Moreover, as shown in Test Example 3 described below, the peptide NLPPLT also has an antidepressive action. The serotonin 5-HT$_{1A}$ receptor is expressed most widely among the various serotonin receptors and is involved in behaviors such as sleep, eating, thermoregulation and anxiety. Accordingly, it has been known that any one or more actions selected from an anxiolytic action, a sleep-improving action and an antidepressive action are generated through activation of the serotonin 5-HT$_{1A}$ receptor. Thus, the peptide as described herein can be used for anxiolytic use, sleep-improving use or antidepressive use.

In addition, the peptide as described herein is believed to be able to prevent, improve or treat various diseases or symptoms related to the serotonin 5-HT$_{1A}$ receptor. Accordingly, the peptide as described herein can be used as an active ingredient for a method for attempting prevention, improvement and/or treatment of a disease, a symptom or the like related to the serotonin 5-HT$_{1A}$ receptor by letting an animal including a human take the peptide or administering the peptide to an animal including a human.

In the present description, the term "improvement" means: improvement of a disease, a symptom or a condition; prevention or delay of deterioration of a disease, a symptom or a condition; or reversal, prevention or delay of progress of a disease or a symptom. The term "prevention" means prevention or delay of the onset of a disease or a symptom in the subject of the application, reduction of the risk of a disease or a symptom of the subject of the application or maintenance of the health.

Examples of the diseases and the symptoms related to the serotonin 5-HT$_{1A}$ receptor include depression, bipolar disorder, schizophrenia, anxiety neurosis, and sleep disorder.

Moreover, because the peptide NLPPLT has an action through activation of the AMPA receptor as shown in Test Example 3 described below, the peptide as described herein is believed to be able to prevent, improve or treat various diseases or symptoms related to the AMPA receptor. It is known that the AMPA receptor is a kind of glutamate receptor, is widely distributed in the central nervous system and is largely involved in memory and learning. Accordingly, it has been known that not only an antidepressive action but also an action of improving learning disorder and/or memory impairment is generated through activation of the AMPA receptor. Thus, the peptide as described herein can be used for anxiolytic use, sleep-improving use or antidepressive use as well as for learning disorder- and/or memory impairment-improving use.

The peptide as described herein is highly safe even with long-term intake and thus can be used as a component of a food or a drink for non-therapeutic purpose.

In the present description, the "non-therapeutic purpose" is a concept which does not include medical practice, namely treatment of a human body by therapy, and examples thereof include beauty treatment, health promotion and the like.

2. Composition

In the present technology, a composition containing the peptide as described herein described above as an active ingredient is also provided. The composition as described herein can be used as a pharmaceutical product such as a preparation, a food or a drink, feed or the like. Moreover, the peptide as described herein can be used for producing the various compositions and the like.

For the composition as described herein, in addition to the peptide as described herein, a component may be used in combination according to the need. As the component, a component which is acceptable for a pharmaceutical product, a food or a drink, feed or the like can be appropriately used.

When the composition as described herein is used for a pharmaceutical product, the pharmaceutical product can be prepared by adding the peptide as described herein to a known pharmaceutical product, or a new pharmaceutical product can also be produced by mixing the peptide in a raw material of the pharmaceutical product.

Moreover, when the peptide as described herein is used for a pharmaceutical product, the peptide may be used directly or used after concentrating or processing into solid, liquid, granules, or powder.

The pharmaceutical product can be appropriately formulated into a desired dosage form depending on the administration method, such as oral administration and parenteral administration. The dosage form is not particularly limited, but in the case of oral administration, for example, the pharmaceutical product can be formulated into a solid preparation such as powder, granules, tablets, troches and capsules, a liquid preparation such as a solution, a syrup, a suspension, and an emulsion or the like. In the case of parenteral administration, for example, the pharmaceutical product can be formulated into a suppository, spray, inhalant, ointment, a plaster, an injection or the like. In the present technology, formulation into a dosage form for oral administration is preferable.

The formulation can be appropriately conducted by a known method depending on the dosage form.

The formulation may also be conducted, for example, by appropriately blending a carrier for formulation. Moreover, in addition to the pharmaceutical product, a component which is generally used for formulation, such as excipients, pH-adjusting agents, colorants and corrigents, can be used. A component having an effect of treating, preventing and/or improving a disease or a symptom which is known or will be found in the future can also be appropriately used in combination.

As the carrier for formulation, various organic or inorganic carriers can be used depending on the dosage form.

Examples of the carrier for a solid preparation include excipients, binders, disintegrating agents, lubricants, stabilizers, and flavoring agents.

Examples of the excipients include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; and sulfate derivatives such as calcium sulfate.

Examples of the binders include, in addition to the excipients, gelatin, polyvinylpyrrolidone, and macrogol.

Examples of the disintegrating agents include, in addition to the excipients, chemically modified starch or cellulose derivatives such as croscarmellose sodium, sodium carboxymethyl starch and cross-linked polyvinylpyrrolidone.

Examples of the lubricants include: talc; stearic acid; metal stearates such as calcium stearate and magnesium stearate; colloidal silica; waxes such as veegum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; and starch derivatives.

Examples of the stabilizers include: paraoxybenzoate esters such as methylparaben and propylparaben; alcohols such as chlorobutanol, benzyl alcohol and phenylethyl alcohol; benzalkonium chloride; acetic anhydride; and sorbic acid.

Examples of the flavoring agents include sweeteners, acidulants, and aromas.

In this regard, the carrier used in the case of a liquid preparation for oral administration is a solvent such as water, a flavoring agent or the like.

When the composition as described herein is used for a food or a drink, the food or the drink can be prepared by adding the peptide as described herein to a known food or drink, or a new food or drink can also be produced by mixing the peptide in a raw material of the food or the drink.

Moreover, when the peptide as described herein is used for a food or a drink, the peptide may be used directly or used after concentrating or processing into solid, liquid, granules or powder.

The food or the drink is not limited regarding the form such as liquid, paste, solid, and powder, and examples thereof include, in addition to tablet candies, liquid foods, feed (including food for pets) and the like, wheat products, instant foods, processed agricultural products, processed fishery products, processed livestock products, milk/dairy products, oils and fats, basic condiments, compound flavor enhancers/foods, frozen foods, confectioneries, drinks, and commercial foods other than those listed above.

Examples of the dairy products include fermented milk, milk beverages, lactic acid bacteria beverages, sweetened condensed milk, skim milk powder, sweetened milk powder, powdered formula, creams, cheeses, butter, and ice creams.

Examples of the wheat products include breads, macaroni, spaghetti, noodles, cake mixes, frying flours, and bread crumbs.

Examples of the instant foods include instant noodles, cup noodles, retort-pouched/prepared foods, prepared canned foods, microwave foods, instant soups/stews, instant miso soups/clear Japanese soups, canned soups, freeze-dried foods, and other instant foods.

Examples of the processed agricultural products include canned agricultural products, canned fruits, jams/marmalades, pickles, cooked beans, dried agricultural products, and cereals (processed grains).

Examples of the processed fishery products include canned fishery products, fish hams/sausages, fishery paste products, fishery delicacies, and *Tsukudani* (foods boiled down in sweetened soy sauce).

Examples of the processed livestock products include canned livestock products/pastes, and livestock hams/sausages.

Examples of the oils and fats include butter, margarine, and vegetable oils.

Examples of the basic condiments include soy sauce, soybean paste, sauces, processed tomato condiments, *Mirin* (sweet sake for seasoning), and vinegars, and the compound flavor enhancers/foods include cooking mixes, curry roux, sauces, dressings, noodle broths, spices, and other compound flavor enhancers.

Examples of the frozen foods include frozen food materials, semi-cooked frozen foods, and cooked frozen foods.

Examples of the confectioneries include caramels, candies, chewing gums, chocolates, cookies, biscuits, cakes, pies, snacks, crackers, Japanese-style confectioneries, rice confectioneries, bean confectioneries, desserts, and other confectioneries.

Examples of the drinks include carbonated drinks, natural juices, fruit juices, fruit juice-containing soft drinks, fruit flesh drinks, fruit granule-containing fruit juices, vegetable drinks, soy milk, soy milk drinks, coffee drinks, tea drinks, drink powders, concentrated drinks, sport drinks, nutritional drinks, alcohols, and other luxury beverages.

Examples of commercial foods other than the foods described above include baby foods, *Furikake* (dry Japanese seasonings), and seasonings for *Chazuke* (boiled rice with hot tea).

Moreover, the food or the drink defined in the present technology can be provided/sold as a food or a drink with a label such as having health benefits (for example, anxiolytic use, sleep-improving use, antidepressive use, learning disorder- and/or memory impairment-improving use or the like).

In the present technology, the "labeling" act includes all the acts for informing a consumer of the use, and all the expressions which can remind or suggest the potential use are the "labeling" acts of this art, regardless of the purposes of labeling, the contents of labeling, the objects to be labeled, the media and the like.

The "label" is preferably an expression which allows a consumer to directly recognize the use. Specific examples include an act of transferring an article in which the use is described on a product regarding the food or the drink or packaging of a product, delivering such an article, displaying such an article for transfer or delivery or importing such an article, and an act of displaying or distributing an advertisement of a product, a price list or a business document with a description of the use thereon or providing information with such contents with a description of the use by an electromagnetic method (for example, internet or the like).

The content of the label is preferably a label approved by the appropriate government agency or the like (for example, a label approved based on a system provided by the administration and performed in the form based on the approval or the like). It is preferable to label with such a content on packaging, a container, a catalogue, a brochure, an advertisement material in a sales site such as POP (Point of purchase advertising), other documents or the like.

The "labels" also include labels with health foods, functional foods, foods for the ill, enteral nutrition products, food for special dietary uses, food with health claims, foods for specified health uses, foods with function claims, foods with nutrient function claims, and quasi-drugs. In particular, the labels are labels approved by the Consumer Affairs Agency, such as labels approved by the systems for foods for specified health uses, the systems for foods with function claims and similar systems thereof. More specific examples include a label with foods for specified health uses, a label with qualified foods for specified health uses, a label with foods with function claims, a label indicating influence on the structure and the function of a body, and a label with reduction of disease risk. Typical examples thereof include labels with food for specified health uses (especially labels with health uses) provided by the Regulations for Enforcement of the Health Promotion Act (Order of the Ministry of Health, Labour and Welfare of Japan, No. 86, April 30, 2003), labels with foods with function claims provided by the Food Labeling Act (Act No. 70 of 2013), and similar labels.

The terms used for the labeling described above are not limited only to the terms such as "anxiolytic use", "sleep-improving use", "antidepressive use" and "learning disorder- and/or memory impairment-improving use", and other terms are of course included in the scope as long as the terms indicate an anxiolytic effect, a sleep-improving effect, an antidepressive effect and a learning disorder- and/or memory impairment-improving effect. The terms can be, for example, labels based on various uses which allow a consumer to recognize an anxiolytic effect, a sleep-improving effect, an antidepressive effect, a learning disorder- and/or memory impairment-improving effect or the like.

When the composition as described herein is used for feed, the feed can be prepared by adding the peptide as described herein to known feed, or new feed can also be produced by mixing the peptide in a raw material of the feed.

Moreover, when the peptide as described herein is used for feed, the peptide may be used directly or used after concentrating or processing into solid, liquid, granules, or powder.

Examples of the raw material of the feed include: grains such as corn, wheat, barley and rye; bran such as oat bran, wheat bran, rice bran and defatted rice bran; food manufacturers' by-products such as corn gluten meal and corn jam meal; animal feed such as defatted powder milk, whey, fish powder and bone meal; yeasts such as brewer's yeast; mineral feed such as calcium phosphate and calcium carbonate; oils and fats; amino acids; and saccharides.

Examples of the form of the feed include feed for pets (for example, pet food or the like), livestock feed, and fish farming feed and the like.

The content of the peptide as described herein in the composition as described herein or the amount thereof used is not particularly restricted but is in the range in which an anxiolytic effect or a sleep-improving effect is efficiently obtained, and the peptide is preferably contained to a degree that allows intake without difficulty.

Moreover, the administration or intake intervals of the composition as described herein and the administration or intake period thereof are not particularly limited.

As described above, because the peptide as described herein is believed to be able to prevent, improve or treat various diseases or symptoms related to the serotonin 5-HT$_{1A}$ receptor, the composition as described herein containing the same as an active ingredient can be used, for example, for treating, preventing and/or improving depression, bipolar disorder, schizophrenia, anxiety neurosis or sleep disorder.

In the present technology, the following constitutions can also be adopted.

[1] Use of the peptide of (a) or (b) below for an anxiolytic composition:

(a) the amino acid sequence of SEQ ID NO: 1; and
(b) an amino acid sequence in which one or several amino acids are substituted, deleted or added in the amino acid sequence of SEQ ID NO: 1.

[2] Use of the peptide of (a) or (b) above for the manufacture of an anxiolytic composition.
[3] Use of the peptide of (a) or (b) above for treatment, prevention and/or improvement of depression, bipolar disorder, schizophrenia, anxiety neurosis or sleep disorder.
[4] A method for treating, preventing and/or improving depression, bipolar disorder, schizophrenia, anxiety neurosis or sleep disorder using the peptide of (a) or (b) above as an active ingredient.
[5] Use of the peptide of (a) or (b) above for a sleep-improving composition.
[6] Use of the peptide of (a) or (b) above for the manufacture of a sleep-improving composition.
[7] Use of the peptide of (a) or (b) above for an antidepressive composition.
[8] Use of the peptide of (a) or (b) above for the manufacture of an antidepressive composition.
[9] Use of the peptide of (a) or (b) above for a composition for improving learning disorder and/or memory impairment.
[10] Use of the peptide of (a) or (b) above for the manufacture of a composition for improving learning disorder and/or memory impairment.
[11] A method for treating, preventing and/or improving any one or more kinds selected from the group consisting of anxiety, sleep disorder, depression, learning disorder and memory impairment using the peptide of (a) or (b) below:

(a) the amino acid sequence of SEQ ID NO: 1; and
(b) an amino acid sequence in which one or several amino acids are substituted, deleted or added in the amino acid sequence of SEQ ID NO: 1.

[12] A method for treating, preventing and/or improving any one or more kinds of disease selected from the group consisting of depression, bipolar disorder, schizophrenia and anxiety neurosis using the peptide of (a) or (b) below:

(a) the amino acid sequence of SEQ ID NO: 1; and
(b) an amino acid sequence in which one or several amino acids are substituted, deleted or added in the amino acid sequence of SEQ ID NO: 1.

Examples

The present technology is explained in further detail below based on Examples.

In this regard, Examples explained below are examples of typical Examplesof the present technology, and the scope of the present technology is not to be construed as being narrowed by the Examples.

Production Example 1: Production of Peptide NLPPLT by Enzymatic Hydrolyzed of Whey Protein

A commercial whey protein concentrate in an amount of 1 kg was reconstituted in 9 kg of purified water, and the solution was pasteurized with a plate-type pasteurizer at 75°C for 15 seconds. Then, the pH of the solution was adjusted to 9.0 by adding sodium hydroxide, and then a protease was added to conduct hydrolysis. Thus, a solution containing NLPPLT was obtained. Then, the enzyme was inactivated, and the solution was concentrated by a general method and dried. Thus, about 1 kg of a powder whey protein hydrolysate product containing the peptide NLPPLT (called "WPH" below) was obtained.

Production Example 2: Chemical Synthesis of Peptide NLPPLT

NLPPLT was chemically synthesized by the F-moc method. After deprotection, purification by reversed-phase HPLC (Waters 1525) and lyophilization were conducted. The molecular weight of the obtained peptide was found to be consistent by LCMS.

Test Example 1: Elevated Plus Maze Test

An elevated plus maze having a plus shape in which two flat plates (arms) of 25 cm × 5 cm were crossed was placed at a height of 50 cm from the floor. One type of the arms is closed arms having transparent walls with a height of 15 cm, and a mouse can walk safely without fear of falling. The other arms are open arms without such walls, and the sides are open. Thus, a mouse which fears a high place does not generally prefer the open arms. Through anxiolytic treatment of a mouse, however, the time of stay in the open arms tends to be longer. Thus, in a confirmation test of an anxiolytic action, the anxiolytic activity becomes higher as the time of stay of a mouse in the open arms is longer or as the number of entries is higher.

<1> Test by WPH Administration

The inventors of the present application first conducted a test regarding the WPH prepared in Production Example 1.

<1-1> Test Method

The WPH prepared in Production Example 1 was administered at a dose of 10 mg, 30 mg or 100 mg per 1 kg body weight to male ddy mice 30 minutes before the test, and 30 minutes later, each mouse was put on the central platform where the open arms crossed the closed arms to conduct the test (WPH administration group, n=6). For five minutes after starting, the total time of stay of the mouse on the open arms (time in open arms), the number of entries into the open arms (visit to open arms) and the total number of entries into the arms (total visits) were recorded.
Moreover, as a control, the same test was conducted using male ddy mice to which the WPH as the test substance was not administered (control group, n=6).
From the obtained results, the proportions (%) of the time of stay on the open arms and the proportions (%) of the number of entries into the open arms were calculated as indicators of the anxiolytic activity, and the differences with a statistical significance were examined by the t-test.

<1-2> Test Results

The test results are shown in Table 1 below.

[Table 1]

[Table 1]

|  | Proportion (%) of Time of Stay on Open Arms | Proportion (%) of Number of Entries into Open Arms |
|---|---|---|
| Control Group | 11.6 | 20.8 |
| WPH (10 mg/kg) Administration Group | 18.9 | 28.4 |
| WPH (30 mg/kg) Administration Group | 30.4* | 32.4 |
| WPH (100 mg/kg) Administration Group | 23.5 | 35.5* |

Mean (n=5-6), Tukey-Kramer test, *P<0.05 vs Control

Because the time of stay on the open arms and the number of entries into the open arms increased through the administration of the WPH, compared to those of the control, it was found that the WPH has an anxiolytic activity.

<2> Test by Peptide NLPPLT Administration

The present inventors focused on the peptide NLPPLT contained in the WPH from the results of <1> above and conducted a test also regarding the peptide.

<2-1> Test Method

The peptide NLPPLT synthesized in Production Example 2 was administered at a dose of 3 μg or 10 μg per 1 kg body weight to male ddy mice 30 minutes before the test, and 30 minutes later, each mouse was put on the central platform where the open arms crossed the closed arms to conduct the test (peptide NLPPLT administration group, n=6). For five minutes after starting, the total time of stay of the mouse on the open arms (time in open arms), the number of entries into the open arms (visit to open arms) and the total number of entries into the arms (total visits) were recorded.
Moreover, as a control, the same test was conducted using male ddy mice to which the peptide as the test substance was not administered (control group, n=6).
Furthermore, to examine the mechanism of action, the same test was conducted using male ddy mice to which 10 mg/kg of an antagonist of the serotonin 5-HT$_{1A}$ receptor, WAY100135 (manufactured by Tocris Bioscience) was orally administered in addition to 10 pg/kg body weight of the peptide NLPPLT synthesized in Production Example 2 (peptide NLPPLT + serotonin antagonist administration group, n=5).
From the obtained results, the proportions (%) of the time of stay on the open arms and the proportions (%) of the number of entries into the open arms were calculated as indicators of the anxiolytic activity, and the differences with a statistical significance were examined by the Tukey-Kramer method.

<2-2> Test Results

The test results are shown in Table 2 and Table 3 below.

[Table 2]

[Table 2]

|  | Proportion (%) of Time of Stay on Open Arms | Proportion (%) of Number of Entries into Open Arms |
|---|---|---|
| Control Group | 20.9 | 26.7 |
| Peptide NLPPLT Administration Group | 33.1 | 35.1 |
| Peptide NLPPLT + Serotonin Antagonist Administration Group | 25.9 | 28.6 |
| Mean (n=6) | | |

[Table 3]

[Table 3]

|  | Proportion (%) of Time of Stay on Open Arms | Proportion (%) of Number of Entries into Open Arms |
|---|---|---|
| Control Group | 15.4 | 22.7 |

(continued)

| | Proportion (%) of Time of Stay on Open Arms | Proportion (%) of Number of Entries into Open Arms |
|---|---|---|
| Peptide NLPPLT (3 pg/kg) Administration Group | 19.9 | 23.4 |
| Peptide NLPPLT (10 pg/kg) Administration Group | 24.4 | 31.7 |
| Mean (n=8) | | |

Because the time of stay on the open arms increased through the administration of the peptide NLPPLT, compared to that of the control, it was found that the peptide NLPPLT has an anxiolytic activity.

On the other hand, when the peptide NLPPLT and the antagonist of the serotonin 5-HT$_{1A}$ receptor were simultaneously administered, the time of stay on the open arms decreased compared to that of the administration of the peptide alone and became a comparable proportion to that of the control. It was thus suggested that the action of the peptide NLPPLT is based on an action through activation of the serotonin 5-HT$_{1A}$ receptor.

Moreover, because the peptide NLPPLT has an action through activation of the serotonin 5-HT$_{1A}$ receptor, it was suggested that the peptide NLPPLT is believed to have not only an anxiolytic action but also a sleep-improving action, which is one of actions based on activation of the serotonin 5-HT$_{1A}$ receptor.

Test Example 2: Open Field Test

An open field test was conducted as a behavior test used for screening an antianxiety drug. The test uses a decrease in the proportion of the time of stay of a mouse in the center of the field (referred to as "time of stay" below) caused by administration of an antianxiety drug. The behavior was observed for five minutes, 30 minutes after the administration. Here, in this open field test, a circular field consisting of a plurality of concentric circles with a common center with different radii was used. The circle closest to the center in the field is considered as the center of the field, and the time in which a mouse placed in the center of the field stays in the center of the field is measured.

<1> Test by Peptide NLPPLT Administration

The inventors of the present application focused on the peptide NLPPLT and conducted a test.

<2> Test Method

The peptide NLPPLT synthesized in Production Example 2 was administered at a dose of 10 pg, 30 μg or 100 μg per 1 kg body weight to male ddy mice 30 minutes before the test, and 30 minutes later, each mouse was put in the center of the field to conduct the test (peptide NLPPLT administration group, n=5). For five minutes after starting, the time of stay was recorded.

Moreover, as a control, the same test was conducted using male ddy mice to which the peptide as the test substance was not administered (control group, n=5-6).

From the obtained results, the proportions (%) of the time of stay were calculated as an indicator of the anxiolytic activity.

<3> Test Results

The test results are shown in Table 4 below.

[Table 4]

[Table 4]

| | Proportion (%) of Time of Stay in Center Circle | Proportion (%) of Number of Entries into Center Circle |
|---|---|---|
| Control Group | 0.3 | 1.5 |
| Peptide NLPPLT (0.01 mg/kg) Administration Group | 0.6 | 1.7 |
| Peptide NLPPLT (0.03 mg/kg) Administration Group | 0.7 | 1.6 |
| Peptide NPPLT (0.1 mg/kg) Administration Group | 0.8 | 2.1 |
| Mean (n=5-6) | | |

Because the time of stay increased through the administration of the peptide NLPPLT, compared to that of the control, it was found that the peptide NLPPLT has an anxiolytic activity.

Test Example 3: Tail Suspension Test

A tail suspension test used for screening an antidepressant was conducted. When a mouse is hung by fixing its tail and thus put under inescapable conditions, it is believed that the mouse first tries to escape and moves but becomes immobile by despair with the time. On the other hand, in a mouse treated with an antidepressant, the time of escaping behavior increases, and the immobile time decreases. That is, when the immobile time decreases, the drug is evaluated to have an antidepressive effect. The behavior was observed for six minutes, 30 minutes after the administration.

<1> Test by WPH Administration

The inventors of the present application first conducted a test regarding the WPH prepared in Production Example 1.

<1-1> Test Method

The WPH synthesized in Production Example 1 was administered at a dose of 1 mg, 10 mg or 100 mg per 1 kg body weight to male ddy mice 30 minutes before the test, and 30 minutes later, the mice were hung by fixing their tails to conduct the test (WPH administration group, n=9-11). For six minutes after starting, the time with immobile behavior was recorded.
Moreover, as a control, the same test was conducted using male ddy mice to which the peptide as the test substance was not administered (control group, n=9-11).
From the obtained results, the times (second) with immobile behavior were calculated as an indicator of the antidepressive activity.

<1-2> Test Results

The test results are shown in Table 5 below.

[Table 5]

[Table 5]

|  | Immobile Time (second) |
|---|---|
| Control Group | 77.4 |
| WPH (10 mg/kg) Administration Group | 55.5 |
| WPH (30 mg/kg) Administration Group | 25.7* |
| WPH (100 mg/kg) Administration Group | 13.0* |
| Mean (n=9-11), Tukey-Kramer test, *P<0.05 vs Control | |

Because the time with immobile behavior decreased through the administration of the WPH, compared to that of the control, it was found that the WPH has an antidepressive activity.

<2> Test by Peptide NLPPLT Administration

The inventors of the present application focused on the peptide NLPPLT and conducted a test.

<2-1> Test Method

The peptide NLPPLT synthesized in Production Example 2 was administered at a dose of 10 μg or 30 μg per 1 kg body weight to male ddy mice 30 minutes before the test, and 30 minutes later, the mice were hung by fixing their tails to conduct the test (peptide NLPPLT administration group, n=5-6). For six minutes after starting, the time with immobile behavior was recorded.

Moreover, as a control, the same test was conducted using male ddy mice to which the peptide as the test substance was not administered (control group, n=5-6).

Furthermore, to examine the mechanism of action, the same test was conducted using male ddy mice to which 10 mg/kg of an antagonist of the AMPA receptor, NBQX (manufactured by Cayman chemical) was orally administered in addition to 10 pg/kg of the peptide NLPPLT synthesized in Production Example 2 (peptide NLPPLT + AMPA receptor antagonist administration group, n=10-12).

From the obtained results, the times (second) with immobile behavior were calculated as an indicator of the antidepressive activity.

<2-2> Test Results

The test results are shown in Table 6 below.

[Table 6]

[Table 6]

|  | Immobile Time (second) |
|---|---|
| Control Group | 88.9 |
| Peptide NLPPLT (0.01 mg/kg) Administration Group | 15.7* |
| Peptide NLPPLT (0.03 mg/kg) Administration Group | 27.1* |
| Mean (n=7-8), Tukey-Kramer test, *P<0.05 vs Control | |

Because the time with immobile behavior decreased through the administration of the peptide NLPPLT, compared to that of the control, it was found that the peptide NLPPLT has an antidepressive activity.

Moreover, the results of the test using the AMPA receptor antagonist were as in Table 7 below.

[Table 7]

[Table 7]

|  | Immobile Time (second) |
|---|---|
| Control Group | 99.0* |
| Peptide NLPPLT Administration Group | 23.0 |
| Peptide NLPPLT + AMPA Antagonist Administration Group | 75.3* |
| AMPA Antagonist Administration Group | 69.8 |
| Mean (n=10-12), Tukey-Kramer test, *P<0.05 vs NLPPLT<br>NLPPLT: 0.01 mg/kg (p.o.), NBQX: 10 mg/kg (i.p.) | |

When the peptide NLPPLT and the antagonist of the AMPA receptor were simultaneously administered, the time with immobile behavior increased significantly compared to that of the administration of the peptide alone. It was thus suggested that the action of the peptide NLPPLT is based on an action through activation of the AMPA receptor. The AMPA receptor is known to be involved in an antidepressive effect, which is consistent with the point that the peptide exhibits an antidepressive action. Moreover, because the AMPA receptor is known to be involved also in memory impairment and learning disorder, it was also suggested that the peptide exhibits a memory impairment-improving action and a learning disorder-improving action.

Industrial Applicability

The peptide according to the present technology can be used for anxiolytic use, sleep-improving use, antidepressive use and learning disorder- and/or memory impairment-improving use. Moreover, a composition containing the peptide as an active ingredient can be used in a wide range of fields of a pharmaceutical product, a food or a drink or the like for reducing anxiety symptoms or psychological stress or for treating, preventing and/or improving depression, bipolar disorder, schizophrenia, anxiety neurosis or sleep disorder.

**Claims**

1. A peptide having the amino acid sequence of (a) or (b) below:

   (a) the amino acid sequence of SEQ ID NO: 1; and
   (b) an amino acid sequence in which one or several amino acids are substituted, deleted or added in the amino acid sequence of SEQ ID NO: 1.

2. The peptide according to claim 1 which has an anxiolytic action.

3. The peptide according to claim 1 which has a sleep-improving action.

4. The peptide according to claim 1 which has an antidepressive action.

5. The peptide according to claim 1 which has an action of improving learning disorder and/or memory impairment.

6. A composition containing the peptide according to any one of claims 1 to 5 as an active ingredient.

7. The composition according to claim 6, wherein the composition is a food or a drink.

8. The composition according to claim 6, wherein the composition is a pharmaceutical product.

9. The composition according to claim 8 which is used for treating, preventing and/or improving depression, bipolar disorder, schizophrenia, anxiety neurosis or sleep disorder.

## INTERNATIONAL SEARCH REPORT

| International application No. |
| --- |
| **PCT/JP2022/013912** |

**A. CLASSIFICATION OF SUBJECT MATTER**

*C07K 7/06*(2006.01)i; *A23L 33/18*(2016.01)i; *A61K 38/08*(2019.01)i; *A61P 25/20*(2006.01)i; *A61P 25/22*(2006.01)i; *A61P 25/24*(2006.01)i; *A61P 25/28*(2006.01)i; *C07K 1/12*(2006.01)n; *C12P 21/06*(2006.01)n
FI: C07K7/06 ZNA; A23L33/18; A61P25/22; A61P25/20; A61P25/24; A61P25/28; A61K38/08; C07K1/12; C12P21/06

According to International Patent Classification (IPC) or to both national classification and IPC

**B. FIELDS SEARCHED**

Minimum documentation searched (classification system followed by classification symbols)

C07K7/06; A23L33/18; A61K38/08; A61P25/20; A61P25/22; A61P25/24; A61P25/28; C07K1/12; C12P21/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2001-136995 A (CALPIS CO., LTD.) 22 May 2001 (2001-05-22)<br>paragraphs [0002], [0010]-[0014], [0033] | 1-9 |
| X | JP 2012-31139 A (CALPIS CO., LTD.) 16 February 2012 (2012-02-16)<br>claims, paragraphs [0011], [0013] | 1-9 |
| X | JP 2020-198791 A (SNOW BRAND MILK PRODUCTS CO., LTD.) 17 December 2020 (2020-12-17)<br>claims, paragraph [0024] | 1-9 |
| X | JP 2017-8104 A (KIRIN CO., LTD.) 12 January 2017 (2017-01-12)<br>paragraphs [0043]-[0051] | 1-9 |
| A | JP 2015-519878 A (PRONUTRIA, INC.) 16 July 2015 (2015-07-16)<br>table 13 | 1-9 |
| A | JP 2016-65038 A (MORINAGA MILK INDUSTRY CO., LTD.) 28 April 2016 (2016-04-28)<br>entire text, all drawings | 1-9 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **11 May 2022** | **24 May 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/013912**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2022/013912**

| Patent document cited in search report | | | Publication date (day/month/year) | Patent family member(s) | | Publication date (day/month/year) |
|---|---|---|---|---|---|---|
| JP | 2001-136995 | A | 22 May 2001 | US 6994987 B1 column1, lines 11-24, column 2, line 28 to column 3, line 34, column 5, lines 60-67 WO 2001/034828 A1 | | |
| JP | 2012-31139 | A | 16 February 2012 | US 2012/0277160 A1 claims, paragraphs [0045], [0051] WO 2011/080947 A1 | | |
| JP | 2020-198791 | A | 17 December 2020 | (Family: none) | | |
| JP | 2017-8104 | A | 12 January 2017 | US 2017/0209520 A1 paragraphs [0085]-[0093] WO 2015/194564 A1 | | |
| JP | 2015-519878 | A | 16 July 2015 | US 2015/0011482 A1 table 13 WO 2013/148325 A1 | | |
| JP | 2016-65038 | A | 28 April 2016 | (Family: none) | | |

Form PCT/ISA/210 (patent family annex) (January 2015)

**REFERENCES CITED IN THE DESCRIPTION**

**Patent documents cited in the description**

- WO 2010087480 A

- WO 2016140277 A